# EUROPEAN PATENT APPLICATION

(11) **EP 2 336 352 A1**
(43) Date of publication of application: **22.06.2011**
(21) Application number: 09811144.6
(22) Date of filing: 04.09.2009
(51) Int. Cl.: C12Q 1/34, G01N 33/53

(54) **BIOSENSOR FOR DETECTING ANTI-HIV ANTIBODIES**

(30) Priority: 04.09.2008 ES 200802551
(71) Applicant: Consejo Superior de Investigaciones Cientificas (CSIC), 28006 Madrid (ES); Universidad Autónoma de Barcelona, 08193 Cerdanyola del Valles, Barcelona (ES)
(72) Inventor: LACZKA, Olivier, E-08193 Bellaterra (Barcelona) (ES); DEL CAMPO, Francisco Javier, E-08193 Bellaterra (Barcelona) (ES); MUÑOZ PASCUAL, Francisco Xavier, E-08193 Bellaterra (Barcelona) (ES); VILLAVERDE CORRALES, Antonio P., E-08193 Bellaterra (Barcelona) (ES); FERRER MIRALLES, Neus, E-08193 Bellaterra (Barcelona) (ES); FERRAZ COLOMINA, Rosa María, E-08193 Bellaterra (Barcelona) (ES)
(74) Representative: Ungria Lopez, Javier
(86) International application number: PCT/ES2009/070368
(87) International publication number: WO 2010/026275

(57) **Abstract**

The present invention provides a biosensor that can detect anti-HIV antibodies in a biological sample, based on the combined use of a genetically modified, allosteric enzyme, and a matrix with microelectrode networks. One of the benefits of this biosensor is its high sensitivity, simplicity on the detection and portability.

SEQUENCE LISTING

<110> Consejo Superior de Investigaciones Cientificas y
      Universidad Autónoma de Barcelona

<120> Biosensor for detecting anti-HIV antibodies

<130> 1641.96

<160> 2

<170> PatentIn version 3.5

<210> 1
<211> 1072
<212> PRT
<213> Artificial sequence

<220>
<223> Sequence of beta-galactosidase enzyme NF795gpC.

<220>
<221> SITE
<222> (3)...(8)
<223> Histidine tail (His-tag).

<220>
<221> SITE
<222> (12)...(18)
<223> Recognizing site of the protease.

<220>
<221> SITE
<222> (790)...(825)
<223> Recognizing site for anti-HIV antibodies

<220>
<221> CHAIN
<222> (1)...(1072)
<223> Modified amino acid

<400> 1

Met Ala His His His His His His Ser Ser Gly Glu Asn Leu Tyr Phe
1               5                   10                  15

Gln Gly Ala Val Val Leu Gln Arg Arg Asp Trp Glu Asn Pro Gly Val
            20                  25                  30

Thr Gln Leu Asn Arg Leu Ala Ala His Pro Pro Phe Ala Ser Trp Arg
        35                  40                  45

Asn Ser Glu Glu Ala Arg Thr Asp Arg Pro Ser Gln Gln Leu Arg Ser
    50                  55                  60

Leu Asn Gly Glu Trp Arg Phe Ala Trp Phe Pro Ala Pro Glu Ala Val
65                  70                  75                  80

Pro Glu Ser Trp Leu Glu Cys Asp Leu Pro Asp Ala Asp Thr Val Val
                85                  90                  95

Val Pro Ser Asn Trp Gln Met His Gly Tyr Asp Ala Pro Ile Tyr Thr
            100                 105                 110

Asn Val Thr Tyr Pro Ile Thr Val Asn Pro Pro Phe Val Pro Ala Glu
        115                 120                 125

Asn Pro Thr Gly Cys Tyr Ser Leu Thr Phe Asn Ile Asp Glu Ser Trp
    130                 135                 140

Leu Gln Glu Gly Gln Thr Arg Ile Ile Phe Asp Gly Val Asn Ser Ala
145                 150                 155                 160

Phe His Leu Trp Cys Asn Gly Arg Trp Val Gly Tyr Gly Gln Asp Ser
                165                 170                 175

Xaa Leu Pro Ser Glu Phe Asp Leu Ser Ala Phe Leu Arg Ala Gly Glu
            180                 185                 190

Asn Arg Leu Ala Val Met Val Leu Arg Trp Ser Asp Gly Ser Tyr Leu
        195                 200                 205

Glu Asp Gln Asp Met Trp Arg Met Ser Gly Ile Phe Arg Asp Val Ser
    210                 215                 220

Leu Leu His Lys Pro Thr Thr Gln Ile Ser Asp Phe Gln Val Thr Thr
225                 230                 235                 240

Leu Phe Asn Asp Asp Phe Ser Arg Ala Val Leu Glu Ala Glu Val Gln
                245                 250                 255

Met Tyr Gly Glu Leu Arg Asp Glu Leu Arg Val Thr Val Ser Leu Trp
            260                 265                 270

Gln Gly Glu Thr Gln Val Ala Ser Gly Thr Ala Pro Phe Gly Gly Glu
        275                 280                 285

Ile Ile Asp Glu Arg Gly Gly Tyr Ala Asp Arg Val Thr Leu Arg Leu
    290                 295                 300

Asn Val Glu Asn Pro Glu Leu Trp Ser Ala Glu Ile Pro Asn Leu Tyr
305                 310                 315                 320

Arg Ala Val Val Glu Leu His Thr Ala Asp Gly Thr Leu Ile Glu Ala
                325                 330                 335

Glu Ala Cys Asp Val Gly Phe Arg Glu Val Arg Ile Glu Asn Gly Leu
            340                 345                 350

Leu Leu Leu Asn Gly Lys Pro Leu Leu Ile Arg Gly Val Asn Arg His
        355                 360                 365

Glu His His Pro Leu His Gly Gln Val Met Asp Glu Gln Thr Met Val
    370                 375                 380

Gln Asp Ile Leu Leu Met Lys Gln Asn Asn Phe Asn Ala Val Arg Cys
385                 390                 395                 400

Ser His Tyr Pro Asn His Pro Leu Trp Tyr Thr Leu Cys Asp Arg Tyr
                405                 410                 415

Gly Leu Tyr Val Val Asp Glu Ala Asn Ile Glu Thr His Gly Met Val
            420                 425                 430

Pro Met Asn Arg Leu Thr Asp Asp Pro Arg Trp Leu Pro Ala Met Ser
        435                 440                 445

Glu Arg Val Thr Arg Met Val Gln Arg Asp Arg Asn His Pro Ser Val
    450                 455                 460

Ile Ile Trp Ser Leu Gly Asn Glu Ser Gly His Gly Ala Asn His Asp
465                 470                 475                 480

Ala Leu Tyr Arg Trp Ile Lys Ser Val Asp Pro Ser Arg Pro Val Gln
                485                 490                 495

Tyr Glu Gly Gly Gly Ala Asp Thr Thr Ala Thr Asp Ile Ile Cys Pro
            500                 505                 510

Met Tyr Ala Arg Val Asp Glu Asp Gln Pro Phe Pro Ala Val Pro Lys
        515                 520                 525

Trp Ser Ile Lys Lys Trp Leu Ser Leu Pro Gly Glu Met Arg Pro Leu
    530                 535                 540

Ile Leu Cys Glu Tyr Ala His Ala Met Gly Asn Ser Leu Gly Gly Phe
545                 550                 555                 560

Ala Lys Tyr Trp Gln Ala Phe Arg Gln Tyr Pro Arg Leu Gln Gly Gly
                565                 570                 575

Phe Val Trp Asp Trp Val Asp Gln Ser Leu Ile Lys Tyr Asp Glu Asn
            580                 585                 590

Gly Asn Pro Trp Ser Ala Tyr Gly Gly Asp Phe Gly Asp Thr Pro Asn
        595                 600                 605

Asp Arg Gln Phe Cys Met Asn Gly Leu Val Phe Ala Asp Arg Thr Pro
    610                 615                 620

His Pro Ala Leu Thr Glu Ala Lys His Gln Gln Gln Tyr Phe Gln Phe
625                 630                 635                 640

Arg Leu Ser Gly Arg Thr Ile Glu Val Thr Ser Glu Tyr Leu Phe Arg
                645                 650                 655

His Ser Asp Asn Glu Phe Leu His Trp Met Val Ala Leu Asp Gly Lys
            660                 665                 670

Pro Leu Ala Ser Gly Glu Val Pro Leu Asp Val Gly Pro Gln Gly Lys
        675                 680                 685

Gln Leu Ile Glu Leu Pro Glu Leu Pro Gln Pro Glu Ser Ala Gly Gln
    690                 695                 700

Leu Trp Leu Thr Val Arg Val Val Gln Pro Asn Ala Thr Ala Trp Ser
705                 710                 715                 720

Glu Ala Gly His Ile Ser Ala Trp Gln Gln Trp Arg Leu Ala Glu Asn
                725                 730                 735

Leu Ser Val Thr Leu Pro Ser Ala Ser His Ala Ile Pro Gln Leu Thr
            740                 745                 750

Thr Ser Gly Thr Asp Phe Cys Ile Glu Leu Gly Asn Lys Arg Trp Gln
        755                 760                 765

Phe Asn Arg Gln Ser Gly Phe Leu Ser Gln Met Trp Ile Gly Asp Glu
    770                 775                 780

Lys Gln Leu Leu Thr Pro Leu Arg Asp Gln Phe Thr Arg Ala Pro Leu
785                 790                 795                 800

Asp Asn Asp Ile Gly Val Gly Ser Gly Ile Lys Gln Leu Gln Ala Arg
                805                 810                 815

Ile Leu Ala Val Glu Arg Tyr Leu Lys Asp Gln Gln Leu Leu Gly Ile
            820                 825                 830

Trp Gly Cys Ser Gly Lys Leu Ile Cys Thr Thr Gly Ser Ser Glu Ala
        835                 840                 845

Thr Arg Ile Asp Pro Asn Ala Trp Val Glu Arg Trp Lys Ala Ala Gly
    850                 855                 860

His Tyr Gln Ala Glu Ala Ala Leu Leu Gln Cys Thr Ala Asp Thr Leu
865                 870                 875                 880

Ala Asp Ala Val Leu Ile Thr Thr Ala His Ala Trp Gln His Gln Gly
                885                 890                 895

Lys Thr Leu Phe Ile Ser Arg Lys Thr Tyr Arg Ile Asp Gly His Gly
            900                 905                 910

Glu Met Val Ile Asn Val Asp Val Ala Val Ala Ser Asp Thr Pro His
        915                 920                 925

Pro Ala Arg Ile Gly Leu Thr Cys Gln Leu Ala Gln Val Ser Glu Arg
    930                 935                 940

Val Asn Trp Leu Gly Leu Gly Pro Gln Glu Asn Tyr Pro Asp Arg Leu
945                 950                 955                 960

Thr Ala Ala Cys Phe Asp Arg Trp Asp Leu Pro Leu Ser Asp Met Tyr
                965                 970                 975

Thr Pro Tyr Val Phe Pro Ser Glu Asn Gly Leu Arg Cys Gly Thr Arg
            980                 985                 990

Glu Leu Asn Tyr Gly Pro His Gln  Trp Arg Gly Asp Phe  Gln Phe Asn
        995                 1000                 1005

Ile Ser  Arg Tyr Ser Gln Gln  Gln Leu Met Glu Thr  Ser His Arg
    1010                 1015                 1020

His Leu  Leu His Ala Glu Glu  Gly Thr Trp Leu Asn  Ile Asp Gly
    1025                 1030                 1035

Phe His  Met Gly Ile Gly Gly  Asp Asp Ser Trp Ser  Pro Ser Val
    1040                 1045                 1050

Ser Ala  Glu Phe Gln Leu Ser  Ala Gly Arg Tyr His  Tyr Gln Leu
    1055                 1060                 1065

Val Trp  Cys Gln
    1070

<210>  2
<211>  1053
<212>  PRT
<213>  Artificial sequence

<220>
<223>  Sequence corresponding to beta-galactosidase enzyme NF795gpC.

<220>
<221>  SITE
<222>  (790)...(825)
<223>  Recognizing site for anti-HIV antibodies

<220>
<221>  CHAIN
<222>  (1)...(1053)
<223>  Modified amino acid.

<400>  2

Val Val Leu Gln Arg Arg Asp Trp Glu Asn Pro Gly Val Thr Gln Leu
1               5                   10                  15

Asn Arg Leu Ala Ala His Pro Pro Phe Ala Ser Trp Arg Asn Ser Glu
            20                  25                  30

Glu Ala Arg Thr Asp Arg Pro Ser Gln Gln Leu Arg Ser Leu Asn Gly
        35                  40                  45

Glu Trp Arg Phe Ala Trp Phe Pro Ala Pro Glu Ala Val Pro Glu Ser
    50                  55                  60

Trp Leu Glu Cys Asp Leu Pro Asp Ala Asp Thr Val Val Val Pro Ser
65                  70                  75                  80

Asn Trp Gln Met His Gly Tyr Asp Ala Pro Ile Tyr Thr Asn Val Thr
                85                  90                  95

Tyr Pro Ile Thr Val Asn Pro Pro Phe Val Pro Ala Glu Asn Pro Thr
            100                 105                 110

Gly Cys Tyr Ser Leu Thr Phe Asn Ile Asp Glu Ser Trp Leu Gln Glu
        115                 120                 125

Gly Gln Thr Arg Ile Ile Phe Asp Gly Val Asn Ser Ala Phe His Leu
    130                 135                 140

Trp Cys Asn Gly Arg Trp Val Gly Tyr Gly Gln Asp Ser Xaa Leu Pro
145                 150                 155                 160

Ser Glu Phe Asp Leu Ser Ala Phe Leu Arg Ala Gly Glu Asn Arg Leu
                165                 170                 175

Ala Val Met Val Leu Arg Trp Ser Asp Gly Ser Tyr Leu Glu Asp Gln
            180                 185                 190

Asp Met Trp Arg Met Ser Gly Ile Phe Arg Asp Val Ser Leu Leu His
        195                 200                 205

Lys Pro Thr Thr Gln Ile Ser Asp Phe Gln Val Thr Thr Leu Phe Asn
    210                 215                 220

Asp Asp Phe Ser Arg Ala Val Leu Glu Ala Glu Val Gln Met Tyr Gly
225                 230                 235                 240

Glu Leu Arg Asp Glu Leu Arg Val Thr Val Ser Leu Trp Gln Gly Glu
                245                 250                 255

Thr Gln Val Ala Ser Gly Thr Ala Pro Phe Gly Gly Glu Ile Ile Asp
            260                 265                 270

Glu Arg Gly Gly Tyr Ala Asp Arg Val Thr Leu Arg Leu Asn Val Glu
        275                 280                 285

Asn Pro Glu Leu Trp Ser Ala Glu Ile Pro Asn Leu Tyr Arg Ala Val
    290                 295                 300

Val Glu Leu His Thr Ala Asp Gly Thr Leu Ile Glu Ala Glu Ala Cys
305                 310                 315                 320

Asp Val Gly Phe Arg Glu Val Arg Ile Glu Asn Gly Leu Leu Leu Leu
                325                 330                 335

Asn Gly Lys Pro Leu Leu Ile Arg Gly Val Asn Arg His Glu His His
            340                 345                 350

Pro Leu His Gly Gln Val Met Asp Glu Gln Thr Met Val Gln Asp Ile
        355                 360                 365

Leu Leu Met Lys Gln Asn Asn Phe Asn Ala Val Arg Cys Ser His Tyr
    370                 375                 380

Pro Asn His Pro Leu Trp Tyr Thr Leu Cys Asp Arg Tyr Gly Leu Tyr
385                 390                 395                 400

Val Val Asp Glu Ala Asn Ile Glu Thr His Gly Met Val Pro Met Asn
                405                 410                 415

Arg Leu Thr Asp Asp Pro Arg Trp Leu Pro Ala Met Ser Glu Arg Val
            420                 425                 430

Thr Arg Met Val Gln Arg Asp Arg Asn His Pro Ser Val Ile Ile Trp
        435                 440                 445

Ser Leu Gly Asn Glu Ser Gly His Gly Ala Asn His Asp Ala Leu Tyr
    450                 455                 460

Arg Trp Ile Lys Ser Val Asp Pro Ser Arg Pro Val Gln Tyr Glu Gly
465                 470                 475                 480

Gly Gly Ala Asp Thr Thr Ala Thr Asp Ile Ile Cys Pro Met Tyr Ala
                485                 490                 495

Arg Val Asp Glu Asp Gln Pro Phe Pro Ala Val Pro Lys Trp Ser Ile
            500                 505                 510

Lys Lys Trp Leu Ser Leu Pro Gly Glu Met Arg Pro Leu Ile Leu Cys
        515                 520                 525

Glu Tyr Ala His Ala Met Gly Asn Ser Leu Gly Gly Phe Ala Lys Tyr
    530                 535                 540

Trp Gln Ala Phe Arg Gln Tyr Pro Arg Leu Gln Gly Gly Phe Val Trp
545                 550                 555                 560

Asp Trp Val Asp Gln Ser Leu Ile Lys Tyr Asp Glu Asn Gly Asn Pro
                565                 570                 575

Trp Ser Ala Tyr Gly Gly Asp Phe Gly Asp Thr Pro Asn Asp Arg Gln
            580                 585                 590

Phe Cys Met Asn Gly Leu Val Phe Ala Asp Arg Thr Pro His Pro Ala
        595                 600                 605

Leu Thr Glu Ala Lys His Gln Gln Gln Tyr Phe Gln Phe Arg Leu Ser
    610                 615                 620

Gly Arg Thr Ile Glu Val Thr Ser Glu Tyr Leu Phe Arg His Ser Asp
625                 630                 635                 640

Asn Glu Phe Leu His Trp Met Val Ala Leu Asp Gly Lys Pro Leu Ala
                645                 650                 655

Ser Gly Glu Val Pro Leu Asp Val Gly Pro Gln Gly Lys Gln Leu Ile
            660                 665                 670

Glu Leu Pro Glu Leu Pro Gln Pro Glu Ser Ala Gly Gln Leu Trp Leu
        675                 680                 685

Thr Val Arg Val Val Gln Pro Asn Ala Thr Ala Trp Ser Glu Ala Gly
    690                 695                 700

His Ile Ser Ala Trp Gln Gln Trp Arg Leu Ala Glu Asn Leu Ser Val
705                 710                 715                 720

Thr Leu Pro Ser Ala Ser His Ala Ile Pro Gln Leu Thr Thr Ser Gly
                725                 730                 735

Thr Asp Phe Cys Ile Glu Leu Gly Asn Lys Arg Trp Gln Phe Asn Arg
            740                 745                 750

Gln Ser Gly Phe Leu Ser Gln Met Trp Ile Gly Asp Glu Lys Gln Leu
        755                 760                 765

Leu Thr Pro Leu Arg Asp Gln Phe Thr Arg Ala Pro Leu Asp Asn Asp
    770                 775                 780

Ile Gly Val Gly Ser Gly Ile Lys Gln Leu Gln Ala Arg Ile Leu Ala
785                 790                 795                 800

Val Glu Arg Tyr Leu Lys Asp Gln Gln Leu Leu Gly Ile Trp Gly Cys
                805                 810                 815

Ser Gly Lys Leu Ile Cys Thr Thr Gly Ser Ser Glu Ala Thr Arg Ile
            820                 825                 830

Asp Pro Asn Ala Trp Val Glu Arg Trp Lys Ala Ala Gly His Tyr Gln
        835                 840                 845

Ala Glu Ala Ala Leu Leu Gln Cys Thr Ala Asp Thr Leu Ala Asp Ala
    850                 855                 860

Val Leu Ile Thr Thr Ala His Ala Trp Gln His Gln Gly Lys Thr Leu
865                 870                 875                 880

Phe Ile Ser Arg Lys Thr Tyr Arg Ile Asp Gly His Gly Glu Met Val
                885                 890                 895

Ile Asn Val Asp Val Ala Val Ala Ser Asp Thr Pro His Pro Ala Arg
            900                 905                 910

Ile Gly Leu Thr Cys Gln Leu Ala Gln Val Ser Glu Arg Val Asn Trp
        915                 920                 925

Leu Gly Leu Gly Pro Gln Glu Asn Tyr Pro Asp Arg Leu Thr Ala Ala
    930                 935                 940

Cys Phe Asp Arg Trp Asp Leu Pro Leu Ser Asp Met Tyr Thr Pro Tyr
945                 950                 955                 960

Val Phe Pro Ser Glu Asn Gly Leu Arg Cys Gly Thr Arg Glu Leu Asn
                965                 970                 975

Tyr Gly Pro His Gln Trp Arg Gly Asp Phe Gln Phe Asn Ile Ser Arg
            980                 985                 990

Tyr Ser Gln Gln Gln Leu Met Glu Thr Ser His Arg His Leu Leu His
        995                 1000                1005

Ala Glu Glu Gly Thr Trp Leu Asn Ile Asp Gly Phe His Met Gly
    1010                1015                1020

Ile Gly Gly Asp Asp Ser Trp Ser Pro Ser Val Ser Ala Glu Phe
    1025                1030                1035

Gln Leu Ser Ala Gly Arg Tyr His Tyr Gln Leu Val Trp Cys Gln
    1040                1045                1050

## Description

The present invention provides a biosensor that can detect anti-HIV antibodies in a biological sample, based on the combined use of a genetically modified, allosteric enzyme, and a matrix with microelectrode networks. One of the benefits of this biosensor is its high sensitivity, simplicity on the detection and portability.

### STATE OF THE PRIOR ART

Exact diagnosis of infectious diseases is a key element in the field of clinical and veterinary medicine. At present, great efforts are devoted to improve existing tests, allowing the emergence of new methods of detection (Kuiken et al., 2003. Curr. Opin, Biotechnol., 14: 641-646), as well as the discovery of new strategies to ensure early detection of infection with better sensitivity, safety and efficacy (Iqbal et al., 2000. Biosens. Bioelectronics., 15: 549-578, Dominguez EA, 1993. J. Clin. Microbiol., 31: 2286-2290; Lennette EH and Smith, 1999. Laboratory Diagnosis of Viral Infections, Informa Health Care). In the past 20 years, Human immunodeficiency virus (HIV) has been one of the most studied virus, and the main target in the development of new direct or indirect analytical processes (McDougal, 2001, AIDS Rev. 3: 183-193) depending on the use of the virus as a target or the antibodies directed against it. In the first case, the viral components (e.g. nucleic acids or proteins) can be detected by antigen ELISA (Schüpbach et al., 2000. Int J. Antimicrob. Agents, 16: 441-445), or chain reaction polymerase (PCR) (Marie Louie, 2000. CMAJ, 163: 301-309; Elnifro et al., 2000. Clin. Microbiol. Rev., 13: 559-570: Ballagi-Pordany, 1993. Vet. Res Commun., 17: 55-72; Niesters, 2004. Clinical Microbiology Infect., 10: 5-11; Hjelle and Busch, 1989. Arch Pathol. Lab Med 113: 975-980). The indirect methods demonstrate the presence of anti-viral antibodies, the most common being those of the type antibody-ELISA, Western blot (Steckelberg and Cockerill, 1998. Mayo Clin. Proc., 63: 373-380) or other immunoassays (Branson, 2007 . Clin. Infect. Dis., 45: S221-S225). The possibility of having available mobile platforms which allow fast detection is particularly needed in geographical areas lacking adequate medical facilities. In this context, biosensors appear to be a promising alternative to conventional methods of analysis (Amano and Cheng, 2005. Anal. Bioanalysts. Chem, 381: 156-164; Bobby Pejcic et al., 2006. Analyst, 131: 1079 -1090). Allosteric enzymes can be used as effective blo-components because its activity is modulated by the specific recognition of target peptides outside the active site, by specific antibodies against these peptides (Villaverde, 2003. FEBS Lett., 554: 169-172). Allosteric sensors can be constructed by protein inserting engineering from the proper selection of permissive sites in the enzyme and a peptide antigen of the pathogen. The allosteric reaction can be followed in enzyme assays and after short reaction times in simple homogeneous assays. This offers exciting possibilities for fast molecular diagnosis and ultra-fast infectious diseases diagnosis.

Among the different enzymes adapted for use as allosteric sensors (Villaverde, 2003. FEBS Lett., 554: 169-172), the β-galactosidase produced by *Escherichia coli* (β-D-galactoside hydrolase, EC 3.2.1.23) is very convenient. This protein is encoded in the IacZ gene and is composed of four sub-units of 116353 Da each, joined together by non-covalent binding (Jacobson et al., 1994, Nature, 369: 761-766) . It has the ability to hydrolyze both lactose and some synthetic analogs.

Recently methods have been described using the β-galactosidase of *Escherichia coli,* NF795gpC, immobilized on activated agarose for the detection of anti-HIV antibodies by measuring the intensity of color that occurs as a consequence of the transformation of the ONPG substrate by action of the allosteric enzyme (Ferraz et al., 2007. Enzyme and Microbial Technology, 41 (4): 492-497).

In the present invention para-aminophenyl β-D-galactopyranoside (PapG) is used, a substrate resulting in the formation of p-aminophenol, an electro-active product, in order to develop a new sensor platform based on microelectrode networks. An important technical advantage of the present invention with respect to the method described by Ferraz et al. (2007) is that the combination of the enzymes described and the microelectrodes networks, provide the new biosensor with greater sensitivity, thereby increasing efficiency in the detection of persons Infected with HIV virus.

In this sense, the present invention presents a solution to the problem of detection of people infected with HIV virus that improves the diagnosis of this infectious disease in speed and specificity while providing a portable detection system that does not require high cost detectors or specialized personnel, such as is the case of current systems mentioned above.

### EXPLANATION OF THE INVENTION

The present invention provides a biosensor for the detection of anti-HIV antibodies in a biological sample, based on the combined use of a genetically modified allosteric enzyme (β-galactosidase) and a matrix with microelectrode networks. Allosteric enzymes have a different specific activity according to their steric conformation. Said conformation is changed by binding the enzyme to a specific allosteric effector. When anti-HIV antibodies bind to the enzyme by means of peptides, which are the modification of the original allosteric enzyme, the 3D conformation positively changes affecting the performance of the active site, stimulating the enzyme activity. A key point of the biosensor presented in this invention is the use of an allosteric enzyme substrate whose product is electroactive, for example, a molecule that can be oxidized on the microelectrode different materials within a pH range suitable for the allosteric enzyme. Another advantage of this biosensor is its high sensitivity, being able to detect trace amounts of product.

The product concentration is an indirect measure of the amount of anti-HIV antibodies present in the biological sample that bind to peptides anchored in the original allosteric enzyme since, as previously mentioned, the binding of the anti-HIV antibodies (exercise as allosteric effectors), favors the enzymatic activity.

Thus, the present invention combines two technological breakthroughs that allow the detection of HIV infection in blood serum samples. A genetically modified allosteric β-galactosidase which responds to the presence of anti-HIV antibodies has been used. Micro-technologies for fabricating microelectrodes networks as electrochemical signal transducer have also been used, generated by the enzymatic reaction product, which is detected by simple systems described below.

Therefore, in a first aspect of the present invention is described a biosensor comprising:
a. an allosteric enzyme modified, at least, by the addition of a peptide recognized by anti-HIV antibodies,
b. a transducer comprising a matrix with microelectrodes networks and
c. a detector of the electrochemical signal.

The term "allosteric enzyme" refers to an enzyme whose activity is regulated by one or several allosteric sites. The allosteric site is a site distinct from the enzyme active site, which binds an effector (called allosteric effector) in a reversible and not covalent way. The binding of this effector modifies the three-dimensional structure of the enzyme and affects the active site configuration, so that in the case of this invention, increases its enzymatic activity.

The peptide or peptides added to the allosteric enzyme should be recognized by any of the anti-HIV antibodies generated by the human or animal organism as a consequence of their infection by HIV virus. The sequence of the peptides defined corresponds to the sequence of the protein recognized by the antibodies generated by the immune system, this protein is called an antigen and the sequence recognized by the antibody is called epitope. These epitopes bind with its antibody in a highly specific interaction that allows the antibodies to identify and bind only their unique antigen. Therefore, the peptides added to the allosteric enzyme correspond to epitopes of HIV virus proteins, usually envelope proteins such as gp41 or gp120, without excluding other possible antigen epitopes.

In addition, the modification of the allosteric enzyme that is part of the biosensor of the present invention may include nucleotides or amino acids (modified or not) additions in the N-terminal and/or C-terminal regions (such as e.g. stop codons) as well as any removal or addition of nucleotides or amino acids (modified or not) in the internal sites of its sequence.

A biosensor combines a biological nature component and a physical-chemical. In the present invention, the biosensor consists of three parts: the biological sensor, the transducer and the detector.

The transducer of the biosensor of the present invention is the component that transforms the appearance of enzymatic product in a signal. The transducer is a matrix formed by microelectrodes networks that converts the biochemical reaction into a quantifiable electrical signal.

The microelectrodes are a powerful and versatile tool in the study of electrochemical processes. The advantages of microelectrodes are diverse, such as its small size, its use in different environments, operation with very small volumes and its ability to detect trace concentrations of various elements. Microelectrode networks are unions of microelectrodes connected in parallel, usually. They have the advantages of microelectrodes and also add another one very important such as the power amplification where the total current is the sum of the current in each of the individual microelectrodes. Microelectrode networks can be presented as an ordered microelectrode network, or a random network and in other arrangements such as microstrips.

Example 2 of the present invention refers to the system used in the manufacture of microelectrodes, specifying the amount of microdisks, its diameter and its orderly arrangement in the matrix.

The amount of microelectrodes present in a network affects the overall magnitude of the observed current. Thus, increasing the number of network elements results in an increase in the recorded current, because the currents collected by each microelectrode are additive. This allows the use of less sensitive instrumentation, which can reduce the costs of instrumental design and the necessary components.

However, more important than the number of elements forming the matrix (since it could be said that the more the better) is trying to ensure the independent behavior of microelectrodes with respect to others, such that they do not shield against each other. This is achieved moving away the microdisks beyond a minimum distance which is dictated by parameters such as experimental time and the diffusion coefficient of the species involved. The distance that molecules will travel can be estimated, roughly, using the following expression: d = square root (D x t), where d is the distance traveled (m), D is the diffusion coefficient of the molecule in question (m² s⁻¹) and t the duration of the experiment (s).

In the present invention, because of dealing with very long times, it might be thought that the diffusion layers of the network microelectrodes are completely overlapping, such that the device would behave almost like a macroelectrode the area of which would be the surface occupied by the microelectrodes network. However, because the mechanism of formation of product that detects our application starts with an initial concentration of zero and the production occurs evenly throughout the solution space, using a microelectrodes network preserves the advantage of having a higher sensitivity against conventional macroelectrodes.

The principle of electrochemical detection is the electric charge transfer between the analyzed substance (product of the reaction of the allosteric enzyme and the substrate) and an electrical conductor using a working electrode This charge transfer (which is the one detected), is due to oxidation or reduction of the product on the working electrode when applying a constant potential with respect to the reference electrode. The electrochemical reaction involves a charge transfer equal and opposite to the auxiliary electrode, which is not taken into account in the study of the behavior of the enzymatic reaction product.

In the present invention, as described in the examples, the following electrodes have been used:
- Working electrode: the microelectrodes network.
- Reference electrode: Ag/AgCl electrode (3M KCI).
- Auxiliary electrode: vitreous carbon rod.

The use of the types of reference and auxiliary electrodes described in this invention does not limit the use of other electrodes that aim at detecting the electrochemical signal generated by the reaction product resulting from the modified allosteric enzyme activity described in other sections.

The detectors of the electrochemical signal can be amperometric or coulometric. The difference between amperometry and coulometry is that amperometry measures the current produced by oxidation or reduction of analyte, could this be partial or total. Coulometry, on the other hand, measures the total charge, i.e. the integral of the current in time. This makes coulometry more sensitive, since the charge always increases linearly with time, while the current may increase or decrease at any moment. Other types of detectors can also be used such as, e.g. potentiometric, conductimetric or capacitometrlc detector.

In a preferred embodiment, the allosteric enzyme comprised in the biosensor of the invention is the β-galactosidase.

This enzyme relates to any natural or artificial β-galactosidase presenting the features of the allosteric enzyme of this invention. The β-galactosidase is an enzyme with hydrolase activity that catalyzes the hydrolysis of β-galactosides to monosaccharides. This enzyme is encoded by the LacZ gene and comprises four subunits joined together by noncovalent bonds. The β-galactosidase has the ability to hydrolyze both lactose and some synthetic analogs which are primarily used for enzyme assays.

In another preferred embodiment, the peptide added to the allosteric enzyme is an epitope of the transmembrane glycoprotein gp41 of HIV virus.

The outer coat of HIV is a lipid envelope derived from the cell membrane. From this coat the viral transmembrane glycoproteins gp41 and the coat glycoproteins gp120 extend out, allowing the binding of HIV to target cells through a recognition area. In this area lies the epitope sequence which binds the antibodies generated by the immune response. HIV infects cells that have in their surface sequences recognized by glycoproteins gp41 and gp120, in this way the virus binds to the cell membrane.

In another preferred embodiment, the biosensor consists of a modified allosteric enzyme selected from the list comprising SEQ ID NO: 1 or SEQ ID NO: 2. The first sequence belongs to the enzyme NF795gpC and the second to the sequence of the enzyme HisNF795gpC. The latter enzyme has been constructed by the addition of a histidine tail and a cleavage site for a tobacco protease at the amino terminal end of NF795gpC.

As shown in the examples, the enzyme corresponding to the sequence SEQ ID NO: 2 does not present a decrease in the activity of the enzyme NF795gpC. Furthermore, the allosteric enzyme selected may include nucleotides or amino acids additions (modified or not) in the N-terminal and/or C-terminal regions as well as any removal or addition of nucleotides or amino acids (modified or not) in internal sites of its sequence.

Another aspect of the present invention is the use of the biosensor for the detection of anti-HIV antibodies in a biological sample.

The term "biological sample" refers to a single sample that can come from a physiological fluid such as blood, plasma, serum or urine and/or any cell tissue from an organism.

For the detection of anti-HIV antibodies in the biological sample is required the addition of a suitable substrate of the allosteric enzyme that allows to indirectly determine the concentration of antibodies bound to the modified allosteric enzyme in the meaning specified in the method described in the next paragraph.

In another aspect of the present invention a method for detecting anti-HIV antibodies is provided, comprising:
a. extracting a biological sample
b. contacting the biological sample from (a) with the biosensor of the invention,
c. adding a substrate of the allosteric enzyme to the sample and the biosensor from (b)
d. recording the electrochemical signal generated in step (c).

Depending on the type of biological sample it will be necessary a different treatment of the same for the next step of this method to be effective. These treatments include the dilutions necessary to increase the effectiveness of the method.

The incubation has to be carried out in an optimal medium for enzyme activity, i.e. with the pH adjusted to optimum conditions in which the enzyme is active as well as the use of buffers to be kept in that range. The incubation should be done with an optimal dilution of the sample (one that allows greater sensitivity in detection), suitable concentration of the modified allosteric enzyme and during the time necessary for the binding of the anti-HIV antibodies to the peptides added to the enzyme to be carried out. In Example 5 of the present Invention the above-mentioned incubation conditions are described, where the dilution of the serum of the samples from patients infected with HIV is 1/320 or 1/60, the enzyme concentration is 2 mg/L and the incubation time is at least 45 minutes.

In chemical reactions involving enzymes, enzyme reactions, the molecules on which the enzyme acts in the beginning of the process are called substrates, and they turn them into different molecules, the products. The substrate binds to the active site of the enzyme, and an enzyme-substrate complex is formed. The substrate is transformed into product by the enzyme action and is released from the active site, becoming free to receive another substrate. The added substrate concentration must not exceed that which does not inhibit the enzymatic action.

As described in the preceding pages, the binding of anti-HIV antibodies to the peptides added to the allosteric protein, stimulates enzyme activity, facilitating the binding of a substrate to the active site of the enzyme. The greater the number of bound antibodies, the more amount of substrate is able to transform. The substrate added in this step should be the one whose product resulting from the enzymatic reaction, is electroactive.

The term "electroactive product" is an electroactive chemical species, i.e. a chemical species in solution that has been formed by a previous chemical reaction from a non-electroactive chemical substance (substrate). This electroactive species can be oxidized or reduced, thereby generating a stream of electrons. The recording of this stream of electrons is carried out with the detection techniques described in the relevant section. In the examples of the present invention, the electroactive product is p-aminophenol (PAP) that generates a stream of electrons when oxidized.

In a preferred embodiment, the substrate of the allosteric enzyme is selected from the list comprising: ONPG (o-nitrophenyl-β-D-galactopyranoside), CPRG (chlorophenyl red-β-D-galactopyranoside), FDG (Fludeoxyglucose also known as fluorescein di-β-D-galactopyranoside) or PAPG (p-aminophenyl-β-D-galactopyranoside).

Another aspect of the present invention is a kit for detecting anti-HIV antibodies comprising the biosensor of the invention.

In a preferred embodiment, the kit also includes an allosteric enzyme substrate, In a more preferred embodiment of the present invention, the allosteric enzyme substrate included in the kit, is selected from the list comprising: ONPG (o-nitrophenyl-β-D-galactopyranoside), CPRG (chlorophenyl red-β-D-galactopyranoside), FDG (Fludeoxyglucose also known as fluorescein di-β-D-galactopyranoside) or PAPG (p-aminophenyl-β-D-galactopyranoside).

Throughout the description and claims the word "comprise" and its variations are not intended to exclude other technical features, additives, components or steps. For those skilled in the art, other objects, advantages and features of the invention will emerge in part from the description and in part from the practice of the invention. The following figures and examples are provided by way of illustration and not intended to be limiting of the present invention,

### DESCRIPTION OF THE FIGURES

FIG 1. Shows the transient currents observed In the microelectrodes network at a constant potential of 0.37 V.
   Measurement of currents in solutions containing the substrate only at 0.25 mg.ml⁻¹ (X), β-galactosidase alone 2 µg.ml⁻¹ (A) or both substrate (0.25 mg.ml⁻¹) and enzyme (2 µg.ml⁻¹) (•) in Z buffer.
FIG 2. Shows the transient currents measured at 0.37 V after one hour of incubation at 28°C.
   The incubation was carried out in the presence of a final dilution 1/60 of serum with anti-HIV antibodies. The substrate concentration was 0.25 mg.ml⁻¹ and 2µg.ml⁻¹ HisNF795gpC in contaminated serum (■) or uninfected (a) and 0.86 µg.ml⁻¹ HisNF795gpC in contaminated (A) or clean (A) serum. Also the measure of the transient current generated by a control with serum but without HisNF795gpC (•) was carried out.

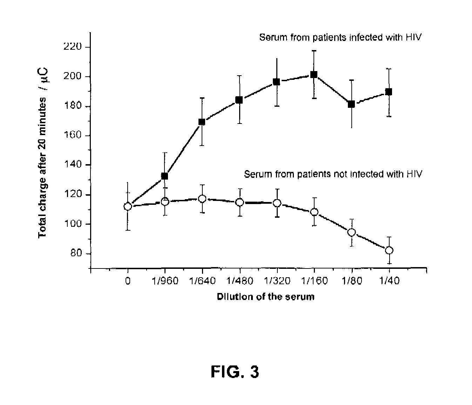

FIG 3. Shows the total passed charge after 20 minutes of applying 0.37 V to the microelectrodes network.
   The signals correspond to the activity of the HisNF795gpC in contact with 0.25 mg.ml⁻¹ of substrate after 45 minutes of incubation at 28°C with positive (■) or negative (a) serum in anti-HIV antibodies.

### EXAMPLES

The following illustrate the invention by means of some tests carried out by the inventors that describe the detection of anti-HIV antibodies.

### EXAMPLE 1

### Cloning, expression, purification and activity assay of recombinant β-galactosidase HisNF795gpC.

The construction of the histidine-labeled recombinant β-galactosidase HisNF795gpC, presenting the epitope B of the envelope protein gp41 or HIV, was based on the addition by PCR of a histidine tail and a cleavage site of the protease of the Tobacco Etch Virus (which allows the removal of histidine) at the NF795gpC amino terminal (Ferrer-Miralles et al., 2001. J. Biol. Chem 276: 40087-40095). The DNA band obtained by PCR (Polimerase Chain Reaction) was cloned into the unique restriction sites Ncol and EcoRI of pNF795gpC. originating in the parental vector pJLA602 (Schauder et al" 1987. Gene, 52: 279-283), resulting in the vector pHisNF795gpC. Said plasmid encodes a modified enzyme under the control of strong promoters of phage lambda pL and pR, repressed, in turn, by the thermosensitive regulator c1857.

The protein HisNF795gpC was produced in E. coli MC4100 using standard methods (Ferrer-Miralles et al., 2001, J, Biol. Chem 276: 40087-40095). The cells were then concentrated by centrifugation and resuspended in Z buffer to which was added 500 mM NaCl, 10 mM imidazole, 2 mM β-mercaptoethanol and tablets of a protease inhibitors cocktail *(Complete EDTA-free from Roche Applied Science).* Z buffer was prepared using PBS lozenges (Invitrogen) to obtain a concentration of 0.1 M PBS and supplementing it with 1 mM MgSO₄ and 20 mM KCI. The solutions were prepared with deionized water of resistivity not less than 18 MΩ cm⁻¹. Bleach was used to clean the material between experiments. The pH of the solutions was monitored using a pH meter *(METROHM 827 pH Lab meter)* with temperature control.

After sonicated and centrifuged, the supernatant was introduced over a nickel column (1 ml HiTrap chelating HP column, GE Healthcare) equilibrated with the same buffer. Then the column was washed with the same buffer and proteins were extracted by a 10-300 mM imidazole gradient. The protease cutting target included after the histidine tail was not used in the purification process because it was not considered necessary since the enzymatic activity of the protein was not affected by the addition of the histidine tail.

Protein positive fractions were detected by a β-galactosidase miniaturized activity assay in ELISA microplates, with ortho-nitrophenyl β-D-galactopyranoside as substrate. The protein fractions collected were dialyzed against buffer Z. The protein concentration was spectrophotometrically estimated by measuring at 260 and 280 nm. The enzyme activity was determined by published variations of the Miller's method (Ferrer-Miralles et al., 2001. J. Biol. Chem 276: 40087-40095).

### EXAMPLE 2

### Characterization of microelectrodes networks.

The microelectrodes networks of thin layer used in this study have already been described elsewhere (Ordeig et al. 2006b. Electroanalysis, 18: 247-252, Ordeig et al. 2006c. Electroanalysis, 18: 573-578). These networks are composed of 128 microdisks of 10 microns in diameter, arranged in a cubic network with an inter-center distance of 100 µm. First, the electrodes were cleaned with ethanol and then were electrochemically activated by applying a series of potential pulses from OV to 2V vs. Ag/AgCl (3M KCl). Then the electrodes were characterized by cyclic voltammetry in 1 mM potassium ferrocyanide. The analysis of limiting currents at different scan rates, such as described in (Ordeig et al., 2006a. Analyst, 131: 440-445), allows the elucidation of the number of active microdisks of the network at any given time throughout the experiments. This operation was repeated after each measurement to determine the degree of passivation of the microelectrodes. Although the performance of the electrodes was usually affected by the adsorption of other proteins present in the suspensions used, it was possible to completely recover the initial behavior of the networks after every cycle of electrochemical activation in a clean electrolyte solution.

### EXAMPLE 3.

### p-aminophenol (PAP) electrochemistry in the microelectrodes networks,

The detection of anti-HIV antibodies was carried out indirectly through the oxidation of p-aminophenol, PAP, which is produced by the β-galactosidase from 4-aminophenyl-β-D-galactopyranoside, PAPG (Niwa et al., 1993. Anal. Chem, 65: 1559-1563; Wollenberger et al., 1994. Analyst, 119: 1245-1249). The advantage of this approach is that the PAP is reversibly oxidized at a moderate potential (ca. 0.3 V vs. Ag/AgCl (3M KCl)) and it virtually does not mess up the electrode (Salavagione et al., 2005. J. Electroanal. Chem, 576: 139-145). However, the PAP is oxidized according to a EC mechanism (Bard and Faulkner, 2000. Electrochemical Methods: Fundamentals and Applications, 2nd Edition, John Wiley & Sons, New York), wherein a first step of electron transfer is produced followed by an associated chemical reaction, moreover, it is known that it is hydrolyzed at a wide range of pH (Wang et al., 1999. J. Electroanal. Chem, 464: 181-186). Furthermore, we found that p-aminophenol was adsorbed weakly on our gold electrodes. This is probably the cause of the range of values found in the literature for the diffusion coefficient of this species (Niwa et al., 1993. Anal. Chem, 65: 1559-1563, Wang et al., 1999. J. Electroanal, Chem, 464: 181-186, Robinson et al., 1990. J. Phys. Chem, 94: 1003-1005). From the analysis of limiting currents obtained in our microelectrodes networks we estimate an apparent diffusion coefficient of (4.45 ± 0.45) x 10-10 m² s⁻¹. We used this value in all calculations of this work. The determination was carried out in Z buffer solution 0.1 M PBS with 1 mM PAP. From the voltammetry we decided to use a working potential of 0.37 V vs. Ag/AgCl (3M KCl) in the experiments of amperometric determination or PAP that followed, since at this potential, the oxidation of PAP is controlled exclusively by diffusion.

The detection limit for the PAP was determined from limiting currents measured at 100 mV s⁻¹. At this scanning speed the diffusional independence of the microdisks in the network is ensured (Davies and Compton, 2005. J. Electroanal. Chem, 585: 63-82, Davies et al., 2005. J. Electroanal. Chem 585: 51-62). The detection limit was estimated at 4 µM from the method of error propagation proposed by Long and Winefordner (1983), as it provides more realistic results than the 3o method of IUPAC (Danzer and Currie, 1998, International Union of Pure and Applied Chemistry, 70: 883-1014).

### EXAMPLE 4

### Electrochemical detection of wild β-galactosidase

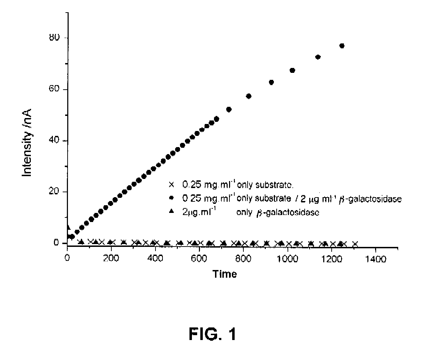

Fig 1 shows that neither the substrate nor the wild β-galactosidase produce a significant response separately, at the work potential and for a polarization time of the electrode of 20 minutes. However, when mixed, the appearance of PAP resulting from the enzymatic activity can be followed with alacrity. For a given concentration of enzyme, the reaction rate is directly related to the oxidation current of the generated product, which in this case is PAP. As the velocity for a given substrate concentration, the initial slope of the curve of current versus time is taken. If the initial velocities measured at a series of substrate concentrations are represented, the value of the constants Km and kcat can be obtained. Kinetics of the enzyme under a classical mechanism of Michaelis-Menten type was studied and values of Km and Kcat of ca. 3.15 x 10⁻⁴ mol l⁻¹ and 0.18 s⁻¹ were respectively found.

### EXAMPLE 5

### Detection of HIV antibodies in serum by His-NF795gpC,

Because His-NF795gpC enzyme is more unstable than the wild-type enzyme, measurements were made in Z buffer containing BSA 1% (weight per volume). However, electrochemical monitoring of enzyme activity continued to be feasible with the microelectrodes networks.

At higher concentrations of the enzyme the current drops after passing through a maximum. As there were no current drops at low levels of enzyme, it was suspected that when the enzyme concentration was too high there was a quantitative consumption of the substrate. Other factors that can affect the sensor response in a similar way are microelectrodes passivation, caused by the adsorption of proteins, but also perhaps the inhibition of the enzyme. Although the enzyme inhibition cannot be ruled out, we think that it is negligible under the experimental conditions of this invention and that the power loss can be explained in general by a combination of substrate consumption and passivation of the electrode.

Immediately after the addition of substrate to the electrochemical cell, the PAP oxidation current is recorded. Incubation of His-NF795gpC protein with positive sera enhanced its activity in at least 56% in all cases studied. In tests carried out with real samples 2 mg L⁻¹ enzyme were incubated for 45 minutes with a 1/60 final dilution of a cocktail of sera from patients infected with HIV-1.

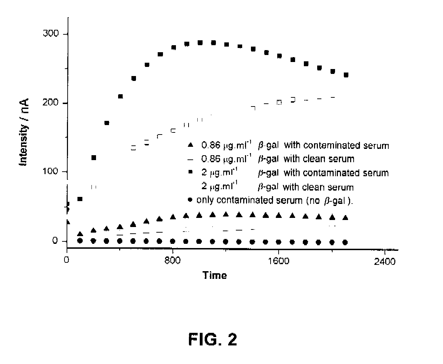

Fig 3 shows the results of the main experiment, which consisted of incubating the enzyme with a bank of serial dilutions of two different types of serum, from individuals infected and healthy individuals, with protein HisNF795gpC. For each condition, three measures that allowed estimating mean values and their corresponding error bars were carried out. We use standard deviations combined with a confidence level of 95% as a measure of error. The recognition of the antibodies was optimal for a 1/320 serum dilution. The increase in the enzyme activity was not enhanced above this level from this point of concentration of serum. Coulometry is a more reliable measure than the measure of the initial velocities of the enzymatic reaction or than measuring the current after 20 min of reaction with the substrate. The charge measurement is more accurate and allows working with lower concentrations of enzyme and substrate (FIG 2).

The potentiostat used in the electrochemical measurements of the examples was an Autolab PG12 controlled with the GPES 4 program and connected to a PC with Windows XP. A 5 cm long, 3 mm in diameter glassy carbon rod was used as auxiliary electrode and a Metrohm Biotrode Ag/Agcl (3M KCI) as reference electrode. The temperature of all solutions was controlled by a double-walled cell connected to a thermostatic bath.

## Claims

1. Biosensor comprising:
a. a modified allosteric enzyme, at least, by the addition of a peptide recognized by anti-HIV antibodies,
b. a transducer comprising a matrix of microelectrodes networks and
c. a detector of the electrochemical signal.

2. Biosensor according to claim 1 wherein the allosteric enzyme is β-galactosidase.

3. Biosensor according to claim 1 wherein the peptide is an epitope of the transmembrane glycoprotein gp41 of HIV virus.

4. Biosensor according to claim 1 wherein the modified allosteric enzyme is selected from the list comprising SEQ ID NO: 1 or SEQ ID NO: 2.

5. Use of the biosensor according to any of claims 1 to 4 for the detection of anti-HIV antibodies in a biological sample.

6. Method for detection of anti-HIV antibodies comprising:
a. extracting a biological sample
b. contacting the biological sample from (a) with the biosensor of the invention,
c. adding a substrate of the allosteric enzyme to the sample and biosensor of step (b) and
d. recording the electrochemical signal generated in step (c).

7. Method according to claim 6 wherein the substrate of the allosteric enzyme is selected from the list comprising: ONPG, CPRG, FDG or PAPG.

8. Kit for the detection of anti-HIV antibodies comprising the biosensor of the invention.

9. Kit according to claim 8 further comprising a substrate of the allosteric enzyme.

10. Kit according to claim 9 wherein the substrate is selected from the list comprising: ONPG, CPRG, FDG or PAPG.
